# DEMANDE DE BREVET EUROPEEN

(11) **EP 2 387 965 A2**
(43) Date de publication de la demande: **23.11.2011**
(21) Numéro de dépôt: 11165859.7
(22) Date de dépôt: 12.05.2011
(51) Int. Cl.: A61B 19/08

(54) **Champ operatoire sous-fessier pour accouchement**

(30) Priorité: 18.05.2010 FR 1053813
(71) Demandeur: Vygon, 95440 Ecouen (FR)
(72) Inventeur: Lestoquoy, Patrick, 59551, ATTICHES (FR)
(74) Mandataire: Le Forestier, Eric

(57) **Abrégé**

L'invention concerne un champ opératoire sous-fessier (1) pour accouchement, destiné à être disposé sur une table opératoire (T) comprenant au moins un étrier (E) adapté pour maintenir en hauteur une jambe de la parturiente et un passage (20) pour l'étrier (E), caractérisé en ce que le passage assure le maintien en position du champ (1) sur l'étrier (E).

## Description

L'invention concerne d'une façon générale le matériel médical, et en l'espèce un champ sous-fessier adapté notamment pour l'accouchement.

Plus particulièrement, l'invention concerne un champ sous-fessier pour accouchement, adapté pour être utilisé que ce soit en position latérale ou obstétricale, en garantissant une position optimale de la parturiente.

Les champs d'accouchement sont des feuilles souples généralement étanches, qui sont placées sur des tables opératoires afin de protéger la table d'une part, et pour le confort des femmes enceintes d'autre part.

L'accouchement est généralement pratiqué en position dite « obstétricale », dans laquelle la parturiente est allongée sur le dos et a les pieds en position surélevée et écartée par rapport à son bassin. Cette position est cependant traumatisante, à la fois pour la parturiente et le bébé.

Il a donc été proposé une nouvelle position, dite latérale, dans laquelle la parturiente est allongée sur le côté, et n'a qu'une seule jambe surélevée par rapport aux hanches, par exemple en appui sur un étrier. On s'est aperçu que cette nouvelle position était moins traumatisante pour la parturiente, et réduisait notamment les risques de déchirement du placenta ainsi que les besoins de réaliser une épisiotomie.

Les champs d'accouchement utilisés conventionnellement ne sont cependant pas adaptés à cette nouvelle position, et ne peuvent protéger de manière efficace l'équipement opératoire ni assurer le confort de la parturiente.

L'invention vise donc à proposer un champ opératoire sous-fessier pour l'accouchement, qui soit à la fois utilisable en cas d'accouchement en position latérale et en cas d'accouchement en position conventionnelle obstétricale.

Pour cela, l'invention propose un champ opératoire sous-fessier pour accouchement, destiné à être disposé sur une table opératoire comprenant au moins un étrier adapté pour maintenir en hauteur une jambe de la parturiente et un passage pour l'étrier, caractérisé en ce que le passage assure le maintien en position du champ sur l'étrier.

Certains aspects préférés mais non limitatifs du champ sont les suivants :
■ des moyens d'étanchéité sont associés au passage pour l'étrier;
■ le passage pour l'étrier est réalisé au niveau de la feuille sous-fessière ;
■ le champ comprend en outre au moins une feuille latérale fixée sur un bord longitudinal de la feuille sous-fessière ;
■ la feuille latérale est fixée de manière amovible à la feuille sous-fessière ;
■ le champ comprend en outre au moins une poche fixée sur la feuille latérale ;
■ le passage est prolongé par un manchon adapté pour être enfilé sur l'étrier ;
■ le manchon comprend des moyens de serrage adaptés pour maintenir le manchon en position sur l'étrier ;
■ une embouchure libre du manchon est fermée de manière étanche par un moyen de fermeture ;
■ le moyen de fermeture de l'embouchure libre du manchon est amovible ;
■ le champ comprend en outre un volet apte à recouvrir le passage pour l'étrier ; et
■ le passage pour l'étrier est un orifice débouchant et présente une dimension telle qu'il est apte à être enfilé sur l'étrier.

Selon un deuxième aspect, l'invention propose un procédé de mise en oeuvre d'un champ d'accouchement conforme à l'invention comprenant les étapes consistant à :
■ placer un champ d'accouchement conforme à l'invention sur une table opératoire ;
■ mettre en position un étrier sur l'un des côtés de la table ; et
■ positionner un élément formant un passage étanche sur l'étrier.

Certains aspects préférés mais non limitatifs du procédé selon l'invention sont les suivants :
■ on place l'élément formant un passage sur l'étrier par emmanchement ;
■ l'élément formant passage est un manchon comprenant un moyen de fermeture amovible au niveau de son embouchure libre, et on ouvre l'embouchure préalablement à la mise en place du manchon sur l'étrier ;
■ si la parturiente est mise en position obstétricale, le procédé comprend en outre les étapes consistant à :
   o mettre en position un deuxième étrier de l'autre côté de la table par rapport au premier étrier ; et
   o emmancher un deuxième élément étanche formant un passage sur le deuxième étrier.
■ le champ comprend en outre une feuille latérale pelable, et le procédé comprend en outre une étape au cours de laquelle on retire la feuille latérale suite à l'accouchement.
■ la feuille sous-fessière comprend des moyens permettant de mesurer un niveau de liquide, par exemple une poche de recueil, et est protégée par un premier pagne et un deuxième pagne, le deuxième pagne étant disposé entre le premier pagne et la feuille de sorte qu'il est protégé lors de l'accouchement, et le procédé comprend en outre les étapes consistant à :
   o retirer le premier pagne pour découvrir le deuxième pagne ; et
   o mesurer le volume de sang perdu par la parturiente.

D'autres caractéristiques, buts et avantages de la présente invention apparaîtront mieux à la lecture de la description détaillée qui va suivre, et en regard des dessins annexés donnés à titre d'exemples non limitatifs et sur lesquels :
- la figure 1a représente une vue du dessus d'un champ d'accouchement conforme à l'invention, en position dépliée et étalée ;
- la figure 1b représente le champ de la figure 1a comprenant en outre une poche de recueil ;
- la figure 2 représente une vue en perspective d'un champ d'accouchement comprenant une première forme de réalisation de manchons et mis en position sur une table d'accouchement ;
- la figure 3 représente un agrandissement d'une section d'un champ d'accouchement comprenant une deuxième forme de réalisation d'un manchon mis en position sur une table d'accouchement ; et
- la figure 4 représente un volet adapté pour être enfilé sur un étrier.

Nous allons décrire à présent un champ opératoire 1 conforme à la présente invention.

Le champ 1 selon l'invention est tout particulièrement adapté à la protection d'une table opératoire T lors d'un accouchement.

Comme illustré sur les figures, un champ 1 conforme à l'invention comprend une feuille sous-fessière 10 réalisée ici en matière plastique, par exemple une résine de synthèse, ou tout autre matériau étanche adapté au domaine médical et chirurgical.

Une extrémité libre de la feuille sous-fessière 10, destinée à être placée en partie haute de la table T (c'est-à-dire du côté de la table T qui est supposé à recevoir la tête de la parturiente), est repliée sur une face avant de la feuille 10 et fixée latéralement à celle-ci pour constituer un rabat 11 sous lequel un opérateur peut glisser les mains pour mettre en place le champ 1 en le poussant sous les fesses de la parturiente.

Le champ 1 comprend en outre au moins un premier pagne 12 fixé sur la face avant de la feuille sous-fessière 10, par exemple au niveau du rabat 11. Par exemple, un bord supérieur du pagne 12 est collé par thermoscellage ou par adhésion sur tout ou partie de la largeur de la feuille sous-fessière 10, sur ou sous la partie repliée du rabat 11, ou encore dans une zone adjacente au rabat 11.

Ce premier pagne 12 sert notamment pour le confort de la parturiente lors de l'accouchement.

Le champ 12 peut en outre comprendre un deuxième pagne 13, fixé sur la feuille de manière à s'étendre sous le premier pagne 12. En variante, le deuxième pagne 13 est fixé sur le premier pagne et peut être détaché de celui-ci. Par exemple, les pagnes sont fixés ensemble de manière amovible au moyen d'un adhésif ou encore par soudage d'une bande intermédiaire spécifiquement pelable (constituée notamment d'au moins deux couches, dont une couche de polyéthylène basse densité).

Le rôle du deuxième pagne 13 est de protéger le champ 1 des pertes sanguines, fluides, etc. provenant de l'accouchement.

Selon un mode de réalisation, le premier 12 et/ou le deuxième pagne 13 sont par exemple réalisés dans un film non tissé, dans un assemblage non tissé, ou dans un voile de polyéthylène, c'est-à-dire dans des matériaux obtenus en assemblant entre elles des fibres par des procédés chimiques ou physiques autres que le tissage, bien connus par ailleurs de la personne de l'art.

Comme illustré sur les dessins, le champ 1 comporte en outre au moins un passage 20 pour un étrier E de la table opératoire T, de préférence deux passages 20. Chaque passage 20 est adapté pour laisser passer un étrier E qui lui est associé à travers le champ 1, de préférence de manière étanche, et permet en outre de maintenir le champ 1 en position par rapport à l'étrier E.

En effet, au cours de l'accouchement, les manipulateurs doivent s'assurer que le champ 1 reste en position sur la table opératoire T, afin de protéger efficacement la parturiente et l'instrumentation stérile, en garantissant que la table T reste propre durant l'accouchement.

Lors d'un accouchement en position obstétricale, l'utilisation des passages 20 n'est pas nécessaire : elle le devient en revanche lors d'un accouchement en position latérale. En effet, dans cette position, un étrier E est installé sur l'un des côtés de la table opératoire T, selon les préférences de la parturiente, afin de soutenir sa jambe et la maintenir en hauteur. On ne fixe en revanche pas d'étrier E de l'autre côté de la table T pour laisser libre passage au personnel médical. L'étrier E qui est utilisé lors de l'accouchement est alors passé à travers le passage 20 qui lui est associé.

Le passage 20 permet alors de protéger la table T en évitant de laisser les fluides (tel que le sang ou le liquide placentaire) s'écouler sur celle-ci.

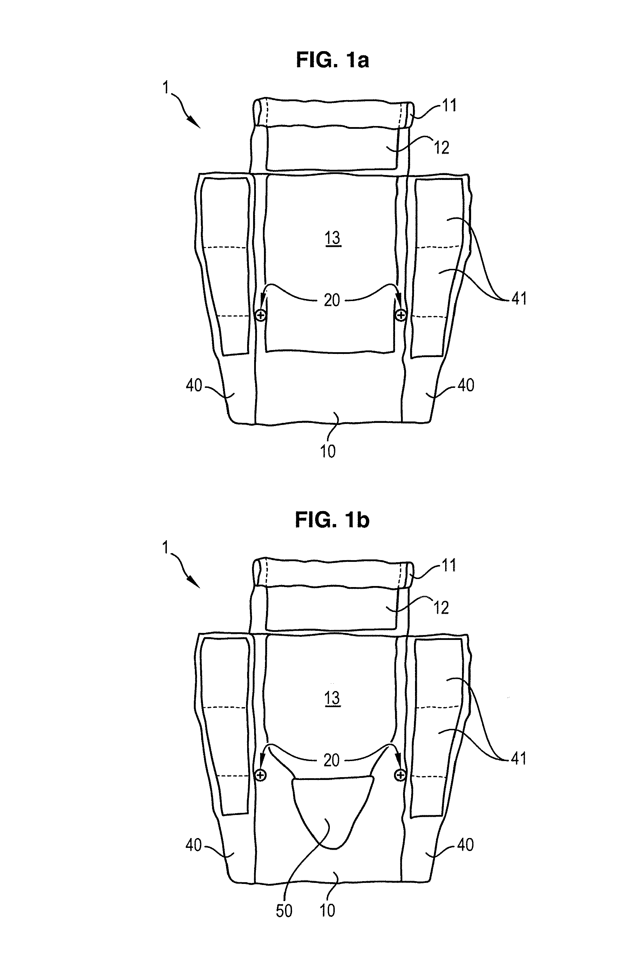

Dans l'exemple de réalisation illustré sur les figures 1a et 1b, le passage 20 est un orifice réalisé dans la feuille sous-fessière 10, à proximité d'un de ses bords longitudinaux, et débouchant sur un espace s'étendant sous le champ, et adapté pour être enfilé sur l'étrier E.

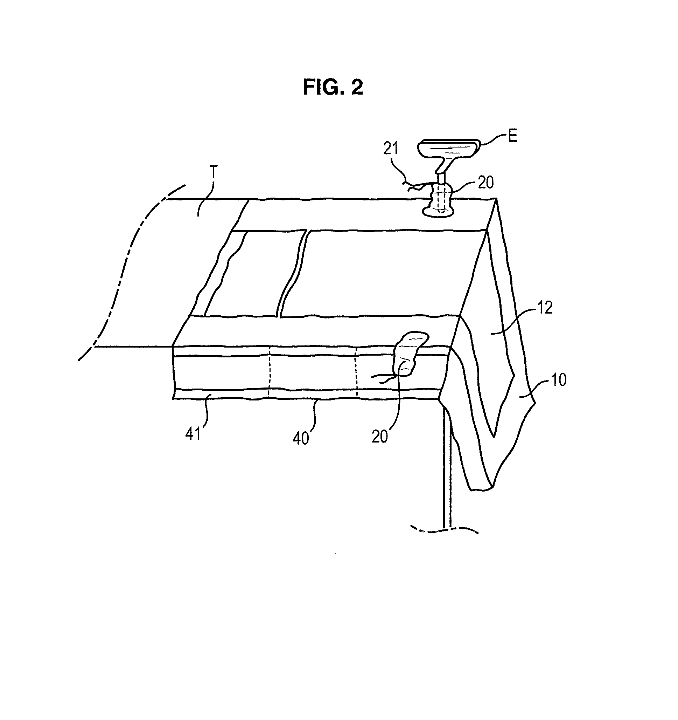

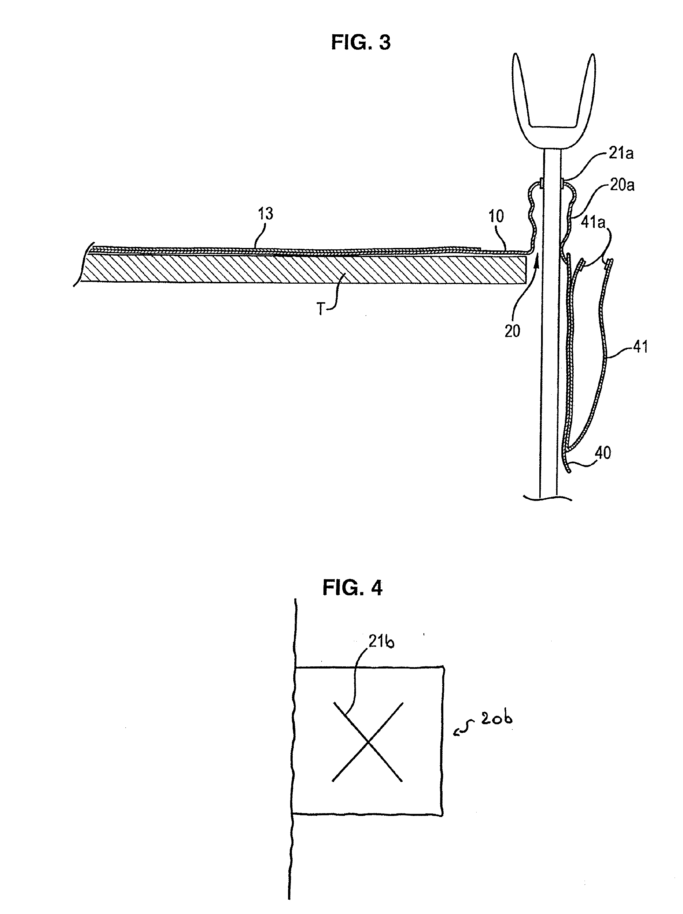

Selon une autre forme de réalisation, illustrée sur les figures 2 et 3, le passage 20 est réalisé dans la feuille sous-fessière 10 ou est constitué par l'espace situé à proximité du bord latéral de la feuille 10, et est prolongé par un manchon 20a adapté pour être emmanché sur l'étrier E. Ici, il est constitué par exemple d'un morceau de tissu réalisé dans le même matériau que celui de la feuille sous-fessière 10 et formant la paroi d'un tube creux. La paroi extérieure du manchon 20a peut alors être fixée sur la face arrière de la feuille 10 ou de manière adjacente à son bord latéral, de sorte que son embouchure inférieure soit ouverte sur l'espace s'étendant sous le champ 1 et soit accessible pour son emmanchement sur l'étrier E qui lui est associé.

Par ailleurs, le manchon 20a peut comprendre des moyens de serrage 21 a de son extrémité libre pour être maintenu en position sur l'étrier E une fois celui-ci enfilé dans le manchon 20a à travers le passage, et éviter que le manchon 20a ne s'affaisse trop en glissant à la base de l'étrier E. Par exemple, il peut s'agir d'un adhésif s'étendant sur le pourtour interne de l'embouchure libre du manchon, d'un lacet, etc.

De préférence, l'embouchure libre des manchons 20 est fermée avant utilisation sur la table opératoire T par une soudure amovible ou tout moyen de fermeture amovible équivalent. Le manchon 20 qui n'est pas utilisé lors de l'accouchement est donc fermé pour éviter qu'il soit traversé par les fluides pouvant salir la table.

En variante, le passage 20 de l'étrier E peut être prolongé par un volet 20b (voir figure 4) s'étendant transversalement depuis l'un des bords longitudinaux de la feuille sous-fessière 10. Le volet 20b est par exemple une feuille plate, qui peut être extensible, présentant deux fentes 21 b en croix adaptée pour venir s'emmancher sur l'étrier E, par exemple par étirement de la feuille 10 et emmanchement en force sur l'étrier E à travers les fentes 21 b. Dans cette forme de réalisation, les fentes 21 b sont par exemple réalisées dans un matériau plastique relativement rigide adapté pour se conformer à l'étrier E lors de l'emmanchement de manière à étanchéifier la fixation du champ 1 sur ledit étrier E et éviter de laisser un passage aux fluides. L'étanchéité du contact peut en outre être renforcée par la mise en oeuvre de moyens adhésifs 21 au niveau des fentes 21 b.

Selon une variante de réalisation, le champ 1 comprend en outre au moins une feuille latérale 40 fixée sur l'un des bords longitudinaux de la feuille sous-fessière.

De préférence, le champ comprend une feuille latérale 40 de chaque côté de la feuille sous-fessière 10. Ces feuilles latérales 40 sont destinées à protéger les côtés longitudinaux de la table opératoire T, adjacents aux étriers E.

Selon une forme de réalisation, les feuilles latérales 40 comportent des poches 41, de préférence étanches, adaptées pour recevoir des fluides (pertes sanguines, liquide physiologique, etc.), des compresses, et tout autre déchet provenant de l'accouchement. Ces poches 41 peuvent éventuellement être amovibles et comporter des moyens de fermeture 41 a en partie haute, par exemple un adhésif, et s'étendre sur tout ou partie de la longueur de la feuille latérale 40.

Avantageusement, les feuilles latérales 40 sont identiques de sorte que le champ d'accouchement 1 soit symétrique et puisse être utilisé indifféremment en position latérale gauche, dans laquelle la parturiente pose sa jambe droite sur l'étrier E à sa gauche, ou en position latérale droite, dans laquelle la parturiente pose sa jambe gauche sur l'étrier E à sa droite.

De préférence, les feuilles latérales 40 sont fixées de manière amovible sur la face arrière de la feuille sous-fessière 10, par exemple par thermoscellage d'un multicouche pelable en polyéthylène (qui peut être la matière du champ 1), par une bande intermédiaire similaire à la bande liant les pagnes, ou encore au moyen d'adhésifs.

Le manchon 20a est alors inséré et fixé entre la feuille sous-fessière 10 et la feuille latérale 40 correspondante (voir notamment figure 3) de sorte que son embouchure inférieure communique avec l'espace s'étendant sous le champ 1 et que l'étrier E puisse être passé dans le manchon depuis ladite embouchure. Dans ce mode de réalisation, le passage 20 est alors constitué par l'espace entre la feuille sous-fessière 10 et la feuille latérale 40 au niveau duquel est fixé le manchon 20a.

De même, dans le cas où le passage 20 est prolongé par le volet 20b, ledit volet 20b peut également être fixé entre la feuille sous-fessière 10 et la feuille latérale 40 correspondante. A nouveau, le passage 20 est constitué ici par l'espace entre la feuille sous-fessière 10 et la feuille latérale 40 au niveau duquel est fixé le volet 20b.

En variante, le volet 20b peut simplement s'étendre depuis la feuille sous-fessière 10. Le passage 20 est alors constitué par l'orifice refermé par les fentes 21 b.

Le champ 1 peut en outre comprendre une poche de recueil 50 étanche et adaptée pour être fixée sur la face avant du champ 1 (c'est-à-dire sur la face de la feuille sous-fessière 1 destinée à recevoir la parturiente, voir figure 1a). La poche 50 est prévue pour recevoir des fluides, le placenta, etc. ainsi que d'autres éléments provenant de l'accouchement, et/ou mesurer les pertes sanguines. Son embouchure est donc orientée vers le haut en utilisation, tandis que son fond est dirigé vers le bas.

Le cas échéant, la poche 50 peut être rapportée ou fixée ab initio sur la feuille sous-fessière 10.

Sur la figure 1b, on a représenté une poche de forme triangulaire fixée sur la feuille sous-fessière, et dans laquelle a été positionné le pagne 13 pour l'accouchement.

Selon une forme de réalisation, la poche 50 est amovible et peut être séparée du champ 1 à tout moment. Elle peut alors comporter des poignées pour faciliter sa manipulation.

Par ailleurs, la poche 50 peut avoir toute forme appropriée, notamment une forme triangulaire, comme illustré sur la figure 1; l'embouchure est alors pratiquée dans un côté du triangle, tandis que le fond est formé par le sommet opposé audit côté.

La poche 50 peut comprendre en outre des moyens adhésifs pour fermer son embouchure de manière étanche.

De telles poches de recueil sont connues en soi et ne feront donc pas l'objet d'une description détaillée.

Nous allons à présent décrire un exemple d'utilisation du champ 1 dans le cadre d'un accouchement.

Dans un premier temps, le champ 1 est placé sur une table opératoire T. L'opérateur peut par exemple placer ses mains dans le rabat 11 et pousser le champ 1 jusqu'à ce qu'il soit en position sur la table T, sous les fesses de la parturiente.

Dans la suite de la description, on décrira la forme de réalisation dans laquelle le champ 1 comprend deux passages 20 prolongés chacun par un manchon 20a, et dans lequel le champ 1 comprend une feuille sous-fessière 10 et deux feuilles latérales 40 amovibles. Ceci n'est cependant pas limitatif : le champ peut ne comprendre qu'un passage 20 et/ou une seule (voire pas de) feuille latérale 40.

Lorsque l'opérateur met en place le champ 1 sur la table opératoire, il emmanche l'un des manchons 20a sur l'étrier E correspondant. Avantageusement, l'étrier E est protégé par une jambière (non illustrée sur les figures), qui peut être rapportée sur l'étrier E et fixée au manchon 20a ou à l'étrier E.

Le choix de l'étrier E et du manchon 20a utilisés est guidé par le confort de la parturiente et n'est pas définitif. En effet, en cours d'accouchement, si celle-ci souhaite se positionner sur l'autre côté que celui sur lequel elle a démarré l'accouchement, il est toujours possible de monter le deuxième étrier E sur la table T et d'emmancher le manchon 20a correspondant sur celui-ci. Ceci est facilité notamment par le fait que le champ 1 comporte les deux manchons 20a dès le début de l'accouchement. On démonte alors l'étrier devenu inutile et on le sort du manchon 20a, afin de libérer l'espace de travail.

La parturiente peut alors s'asseoir sur les pagnes 12, 13 du champ 1 et poser sa jambe sur l'étrier E.

Bien entendu, l'ordre de mise en place mentionné ci-dessus n'est aucunement limitatif. Par exemple, les étriers E peuvent indifféremment être fixés à la table opératoire T avant ou après l'emmanchement des manchons 20a, avant ou après que la parturiente se soit assise sur la table, etc.

Commence alors l'accouchement.

Les différents fluides et déchets sont récupérés par les poches 41 prévues sur les feuilles latérales 40, ou absorbés les cas échéant par la partie centrale de la feuille sous-fessière 10.

A la fin de l'accouchement, les poches 41 sont refermées et éventuellement évacuées, en les détachant des feuilles latérales 40. En variante, les feuilles latérales 40 elles-mêmes sont détachées de la feuille sous-fessière 10 et évacuées avec les poches 41.

La parturiente est alors installée en position obstétricale afin de contrôler le niveau de ses pertes sanguines, par exemple grâce à la poche de recueil 50.

Pour cela, on place le deuxième étrier E sur la table d'opération T à travers le deuxième passage 20 et le deuxième manchon 20a, de manière à maintenir le champ 1 en position sur la table. On fixe alors la poche de recueil 50 sur la partie inférieure du champ (si elle n'y est pas déjà disposée), en contrebas du bassin de la parturiente. Les pertes sanguines survenant suite à l'accouchement sont alors collectées dans la poche 50 et mesurées par transparence grâce à des graduations présentes sur la poche 50.

L'opérateur peut alors contrôler aisément le volume de ces pertes sanguines et déterminer si la parturiente nécessite un traitement médicamenteux d'urgence (conforme par exemple au protocole Exadeli) afin de juguler l'hémorragie.

Si suite à l'opération d'accouchement, il apparaît qu'une suture d'épisiotomie (par exemple) est nécessaire, l'opérateur remplace alors le champ d'accouchement 1 par un champ propre. La parturiente est alors soulevée, tandis que, simultanément, l'opérateur positionne un nouveau champ, puis reposée, et la suture d'épisiotomie peut avoir lieu.

En variante, dans le cas où le champ 1 comprend deux pagnes 12 et 13, la parturiente est soulevée de manière à retirer le premier pagne 12, sali lors de l'accouchement, et découvrir le deuxième pagne 13, protégé par le premier 12 lors de l'accouchement de sorte qu'il peut servir de base propre pour la suite des opérations.

A la fin de l'opération d'épisiotomie, il est alors possible de fermer la poche 50, de la détacher du champ 1 et de l'évacuer.

De la sorte, on garantit à tout moment l'utilisation d'une surface imperméable aux germes sur la table d'opération T au moyen du champ sous-fessier 1, quelle que soit la position de la parturiente lors de l'accouchement, tout en permettant une évacuation étanche et ergonomique des déchets, du placenta, etc.

L'invention permet également de surveiller le niveau des pertes sanguines post-accouchement et d'assurer un nouveau champ propre pour une éventuelle suture d'épisiotomie avec des moyens de mise en place pratiques, ergonomiques et rapides.

## Revendications

1. Champ opératoire sous-fessier (1) pour accouchement, destiné à être disposé sur une table opératoire (T) comprenant au moins un étrier (E) adapté pour maintenir en hauteur une jambe de la parturiente et un passage (20) pour l'étrier (E), **caractérisé en ce que** le passage assure le maintien en position du champ (1) sur l'étrier (E).

2. Champ opératoire (1) selon la revendication précédente, dans lequel des moyens d'étanchéité sont associés au passage (20) pour l'étrier (E).

3. Champ opératoire (1) selon l'une des revendications précédentes, dans lequel le passage (20) pour l'étrier (E) est réalisé au niveau de la feuille sous-fessière (10).

4. Champ opératoire (1) selon l'une des revendications précédentes, comprenant en outre au moins une feuille latérale (40) fixée sur un bord longitudinal de la feuille sous-fessière (10).

5. Champ opératoire (1) selon la revendication précédente, dans lequel la feuille latérale (40) est fixée de manière amovible à la feuille sous-fessière (10).

6. Champ opératoire (1) selon l'une des revendications 4 ou 5, comprenant en outre au moins une poche (41) fixée sur la feuille latérale (40).

7. Champ selon l'une des revendications précédentes, dans lequel le passage (20) est prolongé par un manchon (20a) adapté pour être enfilé sur l'étrier (E).

8. Champ opératoire (1) selon la revendication précédente, dans lequel le manchon (20a) comprend des moyens de serrage (21 a) adaptés pour maintenir le manchon (20a) en position sur l'étrier (E).

9. Champ opératoire (1) selon l'une des revendications précédentes, dans lequel une embouchure libre du manchon (20a) est fermée de manière étanche par un moyen de fermeture.

10. Champ opératoire (1) selon la revendication précédente, dans lequel le moyen de fermeture de l'embouchure libre du manchon (20a) est amovible.

11. Champ opératoire (1) selon l'une des revendications précédentes, comprenant en outre un volet (20b) apte à recouvrir le passage (20) pour l'étrier (E).

12. Champ opératoire (1) selon l'une des revendications précédentes, dans lequel le passage (20) pour l'étrier (E) est un orifice débouchant et présente une dimension telle qu'il est apte à être enfilé sur l'étrier (E).
